(19) Europäisches Patentamt
European Patent Office
Office européen des brevets

(11) EP 1 053 990 A2

(12) EUROPEAN PATENT APPLICATION

(43) Date of publication:
22.11.2000 Bulletin 2000/47

(51) Int. Cl.[7]: **C07C 45/32**, C07C 47/52

(21) Application number: 00304071.4

(22) Date of filing: 15.05.2000

(84) Designated Contracting States:
AT BE CH CY DE DK ES FI FR GB GR IE IT LI LU MC NL PT SE
Designated Extension States:
AL LT LV MK RO SI

(30) Priority: 14.05.1999 JP 13429199

(71) Applicant:
TORAY INDUSTRIES, INC.
Tokyo 103-8666 (JP)

(72) Inventors:
- Nakatani, Jiro
  Aichi 458-0044 (JP)
- Inohara, Masahiro
  Nagoya-shi, Aichi 467-0042 (JP)
- Kato, Tetsuya
  Kanagawa 248-0032 (JP)

(74) Representative:
Coleiro, Raymond et al
MEWBURN ELLIS
York House
23 Kingsway
London WC2B 6HP (GB)

# (54) A method of producing an aromatic ketone and an aromatic ketone composition containing it

(57) In a method of producing an aromatic ketone an aromatic compound having an R-$CH_2$ group ( R denotes an alkyl group, aryl group, allyl group or aralkyl group) is oxidised in liquid phase by an oxygen-containing gas in the presence of a catalyst comprising a heavy metal compound and at least one compound selected from (1) ammonia, (2) organic basic compounds and (3) onium halides while the water produced in the reaction is removed from the reaction solution.

An aromatic ketone composition produced by the process comprises the aromatic ketone and 10 ppm to 3 wt% of at least one compound selected from: a benzene derivative of the formula (II)

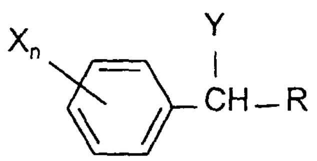

a vinylbenzene of the formula (III)

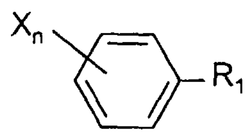

a benzaldehyde of the formula (IV)

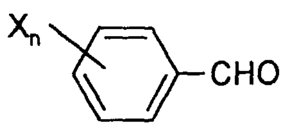

where X is a substituent; n is zero or 1 to 5; R is alkyl, aryl, allyl or aralkyl and $R_1$ is hydrogen, alkyl, aryl, allyl or aralkyl.

EP 1 053 990 A2

## Description

The present invention relates to a method of producing an aromatic ketone, comprising the step of oxidizing an aromatic compound having an R-$CH_2$ group (R denotes an alkyl, aryl group, allyl group or aralkyl group) by oxygen gas in the presence of a catalyst. Aromatic ketones are compounds useful as raw materials of drugs, agricultural chemicals, dyes, perfumes, ultraviolet light absorbers and photographic chemicals.

Known conventional methods for producing an aromatic ketone include a Friedel-Crafts acylation reaction using aluminium chloride as a catalyst. However, according to this method, if the raw material used for reaction has an ortho-para oriented substituent groups, a para product only is produced, and meta and ortho products are little produced. In addition, the method has a large environmental disadvantage that a stoichiometric amount of a Lewis acid, especially aluminum chloride is consumed to produce a large amount of waste after completion of reaction.

On the other hand, the methods of oxidizing an alkylbenzene by air in liquid phase are desired to be established as industrially applicable techniques because of the use of an inexpensive raw material and a low load on the environment. For example, it is known that (A) if ethylbenzene is oxidized in liquid phase in the presence of manganese acetate catalyst, α-phenethyl alcohol and acetophenone are produced (Tadatomo Asaoka, Applied Chemistry, p. 107 (February 10, 1967), Sankyo Shuppan K.K.)(in Japanese). Furthermore, a method of using a transition metal as a catalyst and an onium halide as a co-catalyst, and a method of using a transition metal halide and silver nitrate in combination as a catalyst are respectively disclosed in JP-A-07-196573 and US-A-4042531. Moreover, (B) JP-A-09-278675 and JP-A-09-327626 disclose methods of using an imide compound as a catalyst in combination with a co-catalyst for oxidation in the production of an aromatic ketone, and (C) Journal of Molecular Catalysis A: Vol. 117, p. 159 ~ 168 (1997) discloses a method of oxidizing ethylbenzene using an $Fe_2Ni$ type heteropoly-acid as a catalyst.

However, in attempts to industrially effect the reaction, the method (A) is low in selectivity to produce the intended aromatic ketone, and a benzoic acid cleaved at the bond between α position carbon and β position carbon of the alkyl group is produced in a large amount. Furthermore, the conversion rate of the reaction raw material is also low.

The method (B) is insufficient in selectivity to produce the aromatic ketone, and furthermore since an expensive imide compound is used, it is essential to recover the compound for reuse for industrial application of the method. Even if the compound can be recovered for reuse, a complicated recovery process is necessary.

The method (C) in which an oxidation reaction is effected in the presence of a heteropoly-acid catalyst without any solvent is very low in the yield of the aromatic ketone.

In the production of an aromatic compound with two or more acyl groups, the oxidation of a first R-$CH_2$ group is followed by the oxidation of a second R-$CH_2$ group, and in this case, more severe conditions are required for the second group than that for the first group, and also for the third group than that for the second group. So, the selectivity of the aromatic compound with two or more acyl groups is likely to decrease, and if it is desired to obtain an aromatic compound with two or more acyl groups at a high yield, the catalyst and reaction conditions must be improved further. (D) JP-A-53-053631 discloses a method of producing diacetylbenzene by air oxidation in liquid phase using a molecular oxygen-containing gas in a heterogeneous reaction system consisting of an organic phase containing a reaction substrate and a water phase containing a water soluble catalyst.

However in the method (D), since the water soluble catalyst stays in the water phase, the contact between the reaction substrate in the organic phase and the catalyst is insufficient, and the catalyst does not act so effectively. As a result, the conversion rate of an aromatic compound with two or more R-$CH_2$ groups is low.

None of the above methods is industrially satisfactory.

The present invention addresses the problem of providing a method for efficiently converting an aromatic compound with an R-$CH_2$ group ( R denotes an alkyl group, aryl group, allyl group or aralkyl group) into the corresponding aromatic ketone, using molecular oxygen as an oxidizing agent in the presence of a catalyst.

We made intensive studies in an attempt to solve the above problems, and as a result found that if an aromatic compound having an R-$CH_2$ group (R denotes an alkyl group, aryl group, allyl group or aralkyl group) is oxidized in liquid phase by an oxygen-containing gas in the presence of a catalyst comprising a heavy metal compound and at least one compound selected from (1) ammonia, (2) organic basic compounds and (3) onium halides while the water produced in the reaction is removed from the reaction system, then it can be converted into the corresponding aromatic ketone at a high selectivity.

This invention provides, according to one aspect, a method of producing an aromatic ketone by subjecting an aromatic compound having an R-$CH_2$ group ( R denotes an alkyl group, aryl group, allyl group or aralkyl group) as a reaction raw material to a liquid phase oxidation reaction by an oxygen-containing gas in the presence of a catalyst comprising a heavy metal compound and at least one compound selected from (1) ammonia, (2) organic basic compounds and (3) onium halides while the water produced in the reaction is removed from the reaction system.

Furthermore, according to another aspect this invention provides an aromatic ketone composition comprising an aromatic ketone and at least one compound selected from R-CHY group-substituted benzenes represented by the following formula (II)

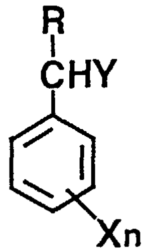

(where X is a substituent; n is zero or an integer of 1 to 5; Y is a hydroxyl group or halogen group and R is as defined above),
vinylbenzenes represented by the following formula (III)

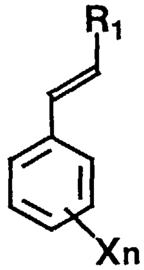

(where X is a substituent; n is zero or an integer of 1 to 5; $R_1$ is a hydrogen atom, alkyl group, aryl group, allyl group or aralkyl group and R is as defined above), and
benzaldehydes represented by the following formula (IV)

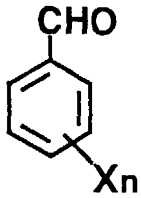

(where X is a substituent; and n is zero or an integer of 1 to 5), each compound of (II), (III) and (IV) present being present individually in an amount of from 10 ppm to 3 wt% by weight of the total weight of the composition.

Preferred embodiments of the invention will now be described.

An aromatic compound having an R-$CH_2$ group ( R is an alkyl group, aryl group, allyl group or aralkyl group) which may be used in this invention is an aromatic compound substituted by one R-$CH_2$ group ( R is an alkyl group, aryl group, allyl group or aralkyl group). An aromatic compound in which at least one substituent group selected from alkyl, aryl, aralkyl, allyl, halogen, haloalkyl, cyano, amino, N-alkylamino, N,N-dialkylamino, alkoxyl, sulfonic acid, hydroxyl, hydroxymethyl, formyl, nitro, acyloxy, acyl, amido, $SR^1$, $SOR^1$, $SO_2R^2$, $CH_2OR^1$, $CH(OR^1)(OR^2)$, $C(OR^1)(OR^2)(OR^3)$ and $COCOR^1$ ($R^1$, $R^2$ and $R^3$ are, respectively independently, an alkyl group, aryl group, allyl group, aralkyl group or hydrogen atom) is further directly bonded to any of the above aromatic rings can also be used as the reaction raw material. As the R of the R-$CH_2$ group, a straight chain alkyl group or branched chain alkyl group with 1 to 8 carbon atoms, allyl group, aryl group with 6 to 15 carbon atoms or aralkyl group with 7 to 16 carbon atoms is preferable. More preferable is alkyl group with 1 to 8 carbon atoms.

For example, an aromatic compound represented by the following formula (I)

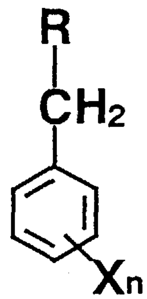

(where X is a substituent; and n is zero or an integer of 1 to 5) can be used.

The aromatic compounds represented by the formula (I) particularly include (each respective hyphenated group named being substituted on an aromatic nucleus) ethyl-benzene, ethyl-toluene, isopropyl-ethyl-benzene, chloro-ethyl-benzene, dichloro-ethyl-benzene, bromo-ethyl-benzene, dibromo-ethyl-benzene, chloro-methyl-ethyl-benzene, dichloro-methyl-ethyl-benzene, trichloro-methyl-ethyl-benzene, fluoro-methyl-ethyl-benzene, difluoromethyl-ethyl-benzene, trifluoro-methyl-ethyl-benzene, nitro-ethyl-benzene, cyano-ethyl-benzene, amino-ethyl-benzene, amido-ethyl-benzene, ethyl-anisole, ethyl-acetophenone, ethyl-propiophenone, ethyl-benzoic acid, ethyl-benzaldehyde, phenyl ethylacetate, hydroxy-ethyl-benzene, propyl-benzene, propyl-toluene, isopropyl-propyl-benzene, chloro-propyl-benzene, dichloro-propyl-benzene, bromo-propyl-benzene, dibromo-propyl-benzene, chloro-methyl-propyl-benzene, dichloro-methyl-propyl-benzene, trichloro-methyl-propyl-benzene, fluoro-methyl-propyl-benzene, difluoro-methyl-propyl-benzene, trifluoro-methyl-propyl-benzene, nitro-propyl-benzene, cyano-propyl-benzene, amino-propyl-benzene, amido-propyl-benzene, propyl-anisole, propyl-acetophenone, propyl-propiophenone, propyl-benzoic acid, propyl-benzaldehyde, phenyl propylacetate, hydroxy-propyl-benzene, n-butyl-benzene, n-butyl-toluene, isopropyl-n-butyl-benzene, chloro-n-butyl-benzene, dichloro-n-butyl-benzene, bromo-n-butyl-benzene, dibromo-n-butyl-benzene, chloro-methyl-n-butyl-benzene, dichloro-methyl-n-butyl-benzene, trichloro-mnethyl-n-butyl-benzene, fluoro-methyl-n-butyl-benzene, difluoro-methyl-n-butyl-benzene, trifluoro-methyl-n-butyl-benzene, nitro-n-butyl-benzene, cyano-n-butyl-benzene, amino-n-butyl-benzene, amido-n-butyl-benzene, n-butyl-anisole, n-butyl-acetophenone, n-butyl-propiophenone, n-butyl-benzoic acid, n-butyl-benzaldehyde, phenyl n-butylacetate, hydroxy-n-butyl-benzene, isobutyl-benzene, isobutyl-toluene, diisobutyl-benzene, isopropyl-isobutyl-benzene, chloro-isobutyl-benzene, dichloro-isobutyl-benzene, bromo-isobutyl-benzene, dibromo-isobutyl-benzene, chloro-methyl-isobutyl-benzene, dichloro-methyl-isobutyl-benzene, trichloro-methyl-isobutyl-benzene, fluoro-methyl-isobutyl-benzene, difluoro-methyl-isobutyl-benzene, trifluoro-methyl-isobutyi-benzene, nitro-isobutyl-benzene, cyano-isobutyl-benzene, amino-isobutyl-benzene, amido-isobutyl-benzene, isobutyl-anisole, isobutyl-acetophenone, isobutyl-propiophenone, isobutyl-benzoic acid, isobutyl-benzaldehyde, phenyl isobutylacetate, hydroxy-isobutyl-benzene, n-pentyl-benzene, n-pentyl-toluene, isopropyl-n-pentyl-benzene, chloro-n-pentyl-benzene, dichloro-n-pentyl-benzene, nitro-n-pentyl-benzene, cyano-n-pentyl-benzene, amino-n-pentyl-benzene, amido-n-pentyl-benzene, n-pentyl-anisole, n-pentyl-acetophenone, n-pentyl-propiophenone, n-pentyl-benzoic acid, n-pentyl-benzaldehyde, phenyl n-pentylacetate, hydroxy-n-pentyl-benzene, diphenylmethane, hydroxy-diphenylmethane, dihydroxy-diphenylmethane, chloro-diphenylmethane, dichloro-diphenylmethane, methoxy-diphenylmethane, dimethoxy-diphenylmethane, methyl-diphenylmethane, dimethyl-diphenylmethane, ethyl-naphthalene, propyl-naphthalene and n-butyl-naphthalene.

Furthermore, this invention allows an aromatic compound substituted by two or more R-$CH_2$ groups to be efficiently converted into an aromatic compound having two or more acyl groups.

The aromatic compounds substituted by two or more R-$CH_2$ groups include, for example, diethyl-benzene, triethyl-benzene, tetraethyl-benzene, methyl-diethyl-benzene, methyl-triethyl-benzene, methyl-tetraethyl-benzene, chloro-diethyl-benzene, chloro-triethyl-benzene, chloro-tetraethyl-benzene, nitro-diethyl-benzene, nitro-triethyl-benzene, nitro-tetraethyl-benzene, bromo-diethyl-benzene, bromo-triethyl-benzene, bromo-tetraethyl-benzene, ethyl-n-propyl-benzene, diethyl-n-propyl-benzene, ethyl-di-n-propyl-benzene, diethyl-di-n-propyl-benzene and di-n-propyl-benzene.

In this invention, when any of the above reaction raw materials is oxidized in the presence of catalyst, it is essential to remove the water produced in the reaction from the reaction system. Unless the water produced in the reaction is removed from the reaction system, it hydrates the heavy metal, and since the reaction raw material cannot reach the heavy metal used as a component of the catalyst, the reaction rate greatly decreases. On the other hand, if the water produced in the reaction is removed from the reaction system, the contact between the reaction raw material and the catalyst occurs sufficiently, allowing the reaction rate and the reaction selectivity to be improved. For removing the produced water, any method can be used. For example, the reaction temperature is set at higher than the boiling point

of water at the reaction pressure, and the water vapor produced by the reaction is condensed by a reflux condenser installed above the reaction solution, and collected to be removed from the reaction system without being allowed to return to the reaction system.

It is preferable that the heavy metal compound used in this invention contains at least one metal selected from groups 3 to 11 of the periodic table. Effective heavy metals include, for example, cobalt, manganese, cerium, vanadium, chromium, tungsten, molybdenum, ruthenium, copper and iron. Especially preferable are cobalt, manganese and cerium.

The heavy metal compound is not especially limited as far as it contains a heavy metal. However, in view of dissolvability in the reaction system and easy availability, inorganic salts such as chlorides, bromides and sulfates and organic salts such as acetates, propionates and acetylacetonates can be exemplified. More preferable are chlorides, bromides and acetates.

It is preferable that the amount of the heavy metal compound used is 0.0001 to 3 wt% as a metal based on the weight of the reaction raw material. A more preferable range is 0.001 to 0.5 wt%. If the amount of the heavy metal compound is 0.0001 wt% or more in the reaction, the oxidation reaction rate can be kept high, and the production selectivity of the intended aromatic ketone can be kept high. If the amount of the heavy metal compound is 3 wt% or less, lowering of the production selectivity of the intended aromatic ketone can be prevented, and no catalyst recovery process is required.

In this invention, a catalyst comprising a heavy metal compound and at least one selected from (1) ammonia, (2) organic basic compounds and (3) onium halides is used.

The organic basic compounds which can be used in this invention refer to organic compounds having a non-shared electron pair and capable of donating it, and include, for example, amine compounds, ether compounds and amide compounds. Among them, amine compounds can be preferably used. Amine compounds can be primary amines, secondary amines and tertiary amines, but tertiary amines are preferable.

Particularly, they include methylamine, dimethylamine, trimethylamine, ethylamine, diethylamine, triethylamine, n-propylamine, di-n-propylamine, tri-n-propylamine, isopropylamine, n-butylamine, isobutylamine, sec-butylamine, tert-butylamine, cyclohexylamine, benzylamine, phenylethylamine, ethylenediamine, aniline, toluidine, anisidine, chloroaniline, cumidine, pyridine, nitropyridine, pyridinesulfonic acid, bromopyridine, chloropyridine, picoline, pyrimidine, pyridazine, pyrazine, pyrazoline, triazine, imidazole, imidazoline, oxazole, oxazoline, thiazole, isothiazole, pyrazole, quinoline, isoquinoline, cinnoline, quinazoline, quinoxaline, phthalazine, purine, pteridine, 1-azaphenanthrene, 1,8-diazaphenanthrene, phenanthridine, acridine, phenazine, 1,10-phenanthroline, nicotinic acid, isonicotinic acid, N,N-dimethylaniline and N,N-diethylaniline.

The onium halides which can be used in this invention include quaternary ammonium halides, quaternary pyridinium halides, oxonium halides, sulfonium halides and quaternary phosphonium halides. The quaternary pyridinium compounds include, particularly, hydropyridinium bromide, hydro-4-dimethylaminopyridinium bromide, hydropicoline bromide, hydropyrimidine bromine and corresponding chlorides and iodides. The quaternary ammonium compounds include hydroammonium bromide, tetramethylammonium bromide, tetraethylammonium bromide, tetrapropylammonium bromide, tetrabutylammonium bromide, benzyltrimethylammonium bromide, 3-methylazolium bromide and corresponding chlorides and iodides. The quaternary phosphonium salts include tetramethylphosphonium bromide, tetraethylphosphonium bromide, tetrapropylphosphonium bromide, tetrabutylphosphonium bromide and corresponding chlorides and iodides. The oxonium halides include $(C_2H_5)_3O^+BF^{4-}$. The sulfonium halides include $(CH_3)_3S^+I^-$.

It is preferable that the total amount of (1) ammonia, (2) organic basic compounds and (3) onium halides used in this invention is 0.01 to 200 molar times the amount of the heavy metal used. A more preferable range is 0.1 to 30 molar times.

In this invention, when the reaction is effected in the presence of (1) ammonia or (2) an organic basic compound, it is preferable to use a halogen compound additionally, since the activity of the catalyst can be promoted. Among halogen compounds, a bromine compound is especially preferable. The bromine compounds which can be used here include inorganic bromine compounds such as hydrogen bromide, alkali metal bromides and ammonium bromide, and organic bromine compounds such as tetrabromoethane, bromoacetic acid and benzyl bromide.

It is preferable that the amount of the halogen compound used in this invention is in a range of 0.05 to 200 molar times the amount of the heavy metal. A more preferable range is 0.5 to 30 molar times. If the halogen compound is used in this range, the equipment can be prevented from being corroded.

In this invention, it is preferable that the reaction system is substantially free from any solvent or if a solvent is used, it is used in an amount of 50 part by weight or less per 100 parts by weight of the reaction raw material. It is more preferable not to use any solvent in the reaction, thereby providing a solvent-less system. This enables the following effects to be achieved, namely that since the reactor efficiency is high, production operation can be effected at high productivity, that it is not necessary to recover the solvent, and that since the reaction temperature can be raised up to the boiling point of the reaction raw material, the reaction can be effected at a high temperature to improve the reaction

rate. Furthermore, surprisingly it has been found that not only the reaction rate but also the aromatic ketone production selectivity can be improved.

The solvents which can be used here include, for example, acetic acid, cyclohexane, benzene, chloroform, carbon tetrachloride, dichloromethane, dichloroethane, diethyl ether, dimethoxyethane, tetrahydrofuran, dioxane, acetone, acetonitrile, nitrobenzene, nitromethane, pyridine, dimethylformamide and dimethyl sulfoxide. If a solvent is used, it is preferable to use a solvent with a high boiling point since the reaction can be effected at a high temperature.

The oxygen-containing gas used in this invention is not required to be pure oxygen, and oxygen diluted, for example, by an inactive gas, such as air can also be used. The required amount of oxygen is a theoretical amount, i.e., 1 mole per one equivalent of the R-$CH_2$ group of the aromatic compound having an R-$CH_2$ group with which the oxygen is caused to react. The gas is usually continuously blown into the reaction system.

The reaction can be effected under reduced pressure, pressurization or atmospheric pressure. Having regard to reaction efficiency (per unit volume), it is not preferable to execute the reaction at a very low pressure. Furthermore, having regard to economy of equipment such as reactor, it is not preferable either to execute the reaction at a very high pressure. A preferable pressure range is 0.01 to 20 MPa, and a more preferable range is 0.05 to 10 MPa. Furthermore, it is preferable that operation is effected to ensure that the oxygen concentration in the exhaust gas from the reactor is kept at lower than the explosion limit concentration.

It is preferable that the reaction temperature is 50 to 300°C. A more preferable range is 80 to 250°C. If the reaction is effected at a temperature of 50°C or higher, the conversion of the aromatic compound having an R-$CH_2$ group (R is an alkyl group, aryl group, allyl group or aralkyl group) as the reaction raw material can be kept high. In other words, the decrease of the reaction rate can be prevented to improve the productivity of the reaction product. Furthermore, if the reaction is effected at a temperature of 300°C or lower, undesirable side reactions can be prevented to suppress the increase of byproducts, and in addition, such a reaction temperature is preferable also having regard to the stability of the aromatic compound having an R-$CH_2$ group (R is an alkyl group, aryl, group, allyl group or aralkyl group) as the reaction raw material, and also having regard to the stability of the produced aromatic ketone. In this case, the reaction selectivity is not lowered.

As the reactor used in this invention, any reactor equipped with forced mixing is preferable to a simple bubble tower reactor. That is, in order to promote the dissolution of the oxygen-containing gas into the reaction solution and to smoothly achieve the mutual contact between reacting materials in the reactor, it is preferable to use a reactor with many gas blow-in ports at the lower portion of the reactor for forced stirring by revolving stirring blades, or a reactor with a circulating pump provided outside the reactor for forced circulation.

A reflux condenser is provided above the reactor, to discharge the exhaust gas through the reflux condenser, for condensing the solvent, reaction raw material, etc. contained in the exhaust gas, for circulation of them in the reactor. In this case, the water produced in the reaction is separated and discharged outside the reaction system.

The aromatic ketone obtained according to this invention can be separated and refined by ordinary distillation, crystallization or chromatography, etc. If the unreacted reaction raw material and the 1-hydroxylalkylbenzene as a precursor of the aromatic ketone are recovered, they can also be reused for the oxidation reaction.

The aromatic ketone obtained according to this method is characterized by containing 10 ppm to 3 wt% of at least one compound selected from R-CHY group substituted benzenes represented by the following formula (II)

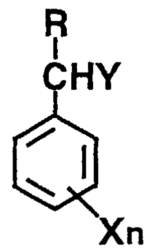

(where X is a substituent; n is zero or an integer of 1 to 5; and Y is a hydroxyl group or halogen atom), vinylbenzenes represented by the following formula (III)

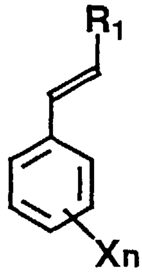

(where X is a substituent group; n is zero or an integer of 1 to 5; and $R_1$ is a hydrogen atom, alkyl group, aryl group, allyl group or aralkyl group), and
benzaldehydes represented by the following formula (IV)

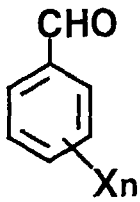

(where X is a substituent; and n is zero or an integer or 1 to 5). It is more preferable that any such compound present is present in an amount of 50 ppm to 2 wt% by weight of the total composition. In the above formulae, the substituent X is the same as the substituent X of the reaction raw material.
Such an impurity is peculiar to the aromatic ketone produced according to the method of this invention. If the method of this invention is used for production, the aromatic ketone obtained contains the impurity. The aromatic ketone produced according to any other method does not contain the impurity in the amount as specified above. In particular, it is peculiar to the aromatic ketone produced according to the method of this invention that the aromatic ketone contains a vinylbenzene represented by the formula (III) or a benzaldehyde represented by the above formula (IV) in the amount specified above.

Preferred embodiments of this invention are now described below in more detail with reference to Examples.

The heavy metal compounds used as catalysts in the following Examples were cobalt chloride hexahydrate produced by Katayama Kagaku, cobalt acetate tetrahydrate produced by Nacalaitesque and cobalt bromide produced by Katayama Kagaku. The tertiary amine used was pyridine produced as a guaranteed reagent by Kishida Kagaku. The halogen compound used was hydrobromic acid (47% HBr aqueous solution) produced by Katayama Kagaku.

The organic acid solvent used was acetic acid produced as a first class reagent by Kanto Kagaku.

The aromatic compounds having an R-$CH_2$ group used were m-chloroethylbenzene (mCEB), p-diethylbenzene (pDEB) and m-diethylbenzene (mDEB) respectively produced by Toray Industries, Inc., and m-chloropropylbenzene (mCPB) synthesized as described below.

(Synthesis of mCPB)

Meta-CPB was prepared from m-chloropropiophenone (mCPP) by Wolff-Kishner reduction.

A 10-liter separable flask equipped with an air cooled fractional distillation column, mechanical stirrer and thermometer was charged with 980 g of mCPP (5.8 mol, produced by Avocado), 1.1 kg of diethylene glycol (produced by Katayama Kagaku), 600 g of potassium hydroxide (10.7 mol, produced by Katayama Kagaku) and 600 g of 80% hydrazine hydrate (9.6 mol, produced by Katayama Kagaku), and with stirring, the temperature of the mantle heater was raised to 120°C. After the temperature of the emission gas became stable (production of hydrazone), the temperature was further raised till the temperature of the emission gas reached 110°C. At the temperature, reaction was effected for about 8 hours.

It was confirmed by GC analysis that the hydrazone vanished, and water was added, to allow the reaction mixture to cool. In succession, mCPB was extracted with hexane, washed with water again and refined by distillation under reduced pressure.

Example 1

A glass three-neck flask with a reflux condenser tube and a Dean-Stark device was charged with 80 g of m-chloroethylbenzene (mCEB), and into the flask, 0.0135 g of cobalt chloride hexahydrate and 0.0045 g of pyridine were supplied. With stirring by stirring blades, air was blown in at atmospheric pressure at 150 ml/min. In succession, the flask was heated by an oil bath to 120°C, to initiate reaction. The water produced in the reaction was removed from the reaction solution by the Dean-Stark device. The organic material boiled with the produced water was returned to the reaction solution after completion of reaction. After reaction for a predetermined period of time, the reaction solution was analyzed by gas chromatography (Table 1) and high performance liquid chromatography. The m-chloroethylbenzene conversion, m-chloroacetophenone yield, 1-(3-chlorophenyl)-ethanol yield, m-chlorobenzoic acid yield and m-chlorobenzaldehyde yield were calculated from the following formulae respectively.

Reaction results are shown in Table 2.

m-chloroethylbenzene conversion (%) =
(Moles of reacting m-chloroethylbenzene)/(Moles of m-chloroethylbenzene in raw materials) x 100

m-chloroacetophenone yield (%) =
(Moles of produced m-chloroacetophenone)/(Moles of m-chloroethylbenzene in raw materials) x 100

1-(3-chlorophenyl)ethanol yield (%) =
(Moles of produced 1-(3-chlorophenyl)ethanol)/(Moles of m-chloroethylbenzene in raw materials) x 100

m-chlorobenzoic acid yield (%) =
(Moles of produced m-chlorobenzoic acid)/(Moles of m-chloroethylbenzene in raw materials) x 100

m-chlorobenzaldehyde yield(%) =
(Moles of produced m-chlorobenzaldehyde)/(Moles of m-chloroethylbenzene in raw materials) x 100

Table 1

| GC analysis conditions | |
|---|---|
| Model | Shimadzu GC-14B |
| Column | NEUTRA BOND-1 produced by GL Science (Capillary Cat. No. AG-1163) 60 m x 0.25 mm, ID 0.4 μm |
| Column temperature (heating pattern) | Initial 100°C (held for 15 min) 3°C/min (up to 250°C, taking 30 min) Final 250°C (held for 25 min) |
| INJ/DET temperature | 250°C |
| Detector | FID |
| Makeup gas | 40 ml/min He |
| Hydrogen | 30 ml/min |
| Air | 400 ml/min |

Sample: Neat 0.4 μl was injected.

Comparative Example 1

An experiment was performed as described for Example 1, except that the produced and condensed water was returned to the reaction system using the reflux condenser tube without using a Dean-Stark device. The results are shown in Table 3.

Example 2

An experiment was performed as described for Example 1, except that 0.0142 g of cobalt acetate tetrahydrate was used instead of 0.0135 g of cobalt chloride hexahydrate, and that the amount of pyridine was changed from 0.0045 g to 0.010 g. The reaction results are shown in Table 2.

Example 3

An experiment was performed as described for Example 1, except that the amount of pyridine was changed from 0.0045 g to 0.09 g, and that 0.196 g of hydrobromic acid aqueous solution (47% HBr) was further added. The reaction results are shown in Table 2.

Example 4

An experiment was performed as described for Example 3, except that the amount of cobalt chloride hexahydrate was changed from 0.0135 to 0.008 g, that the amount of pyridine was changed from 0.09g to 0.08g, that the amount of an aqueous solution of hydrobromic acid (47% HBr) was changed from 0.196g to 0.17g, and that the reaction temperature was changed from 120°C to 140°C. The reaction results are shown in Table 2.

Example 5

An experiment was performed as described for Example 3, except that 0.106 g of α-picoline was used instead of 0.09 g of pyridine, and that the reaction temperature was changed from 120°C to 140°C. The reaction results are shown in Table 2.

Example 6

An experiment was performed as described for Example 1, except that 0.0113 g of 1,10-phenanthroline was used instead of 0.0045 g of pyridine. The reaction results are shown in Table 2.

Example 7

An experiment was performed as described for Example 3, except that 0.0212 g of cerium chloride heptahydrate was used instead of 0.0135 g of cobalt chloride hexahydrate, and that the reaction temperature was changed from 120°C to 140°C. The reaction results are shown in Table 2.

Example 8

An experiment was performed as described for Example 3, except that 0.0113 g of manganese chloride tetrahydrate was used instead of 0.0135 g of cobalt chloride hexahydrate. The reaction results are shown in Table 2.

Comparative Example 2

A glass three-neck flask with a reflux condenser tube but without a Dean-Stark device was charged with 40 g of m-chloroethylbenzene (mCEB) and 120 g of acetic acid, and into the flask, 0.004 g of cobalt chloride hexahydrate, 0.04 g of pyridine and 0.085 g of an aqueous solution of hydrobromic acid were supplied. With stirring by stirring blades, air was blown in at atmospheric pressure at 150 ml/min. In succession, the flask was heated by an oil bath to 110°C, to initiate reaction. After reaction for a predetermined period of time, the reaction solution was analyzed by gas chromatography and high performance liquid chromatography. The results are shown in Table 3.

Comparative Example 3

An experiment was performed as described for Comparative Example 2, except that the amounts of cobalt chloride hexahydrate, pyridine and aqueous solution of hydrobromic acid were increased to 0.016g. 0.16 g and 0.34 g respectively. The results are shown in Table 3.

Comparative Example 4

A glass three-neck flask with a reflux condenser tube without a Dean-Stark device was charged with 14.1 g of m-chloroethylbenzene (mCEB) and 120 g of acetic acid, and into the flask, 0.0872 g of cobalt bromide hexahydrate, 0.117 g of cobalt acetate tetrahydrate and 0.0381 g of sodium acetate were supplied. With stirring by stirring blades, air was blown in at atmospheric pressure at 150 ml/min. In succession, the flask was heated by an oil bath to 110°C, to initiate reaction. After reaction for a predetermined period of time, the reaction solution was analyzed by gas chromatography and high performance liquid chromatography. The results are shown in Table 3.

Comparative Example 5

A glass three-neck flask with a reflux condenser tube and a Dean-Stark device was charged with 80 g of m-chloroethylbenzene (mCEB), and into the flask, 0.0135 g of cobalt chloride hexahydrate was supplied. With stirring by stirring blades, air was blown in at atmospheric pressure at 150 ml/min. In succession, the flask was heated by an oil bath to 120°C, to initiate reaction. The water produced in the reaction was removed from the reaction solution by the Dean-Stark device. The organic material boiled with the produced water was returned to the reaction solution after completion of reaction. After reaction for a predetermined period of time, the reaction solution was analyzed by gas chromatography and high performance liquid chromatography. The results are shown in Table 3.

Comparative Example 6

An experiment was performed as described for Comparative Example 5, except that cobalt acetylacetonate monohydrate was used instead of 0.0135 g of cobalt chloride hexahydrate. The results are shown in Table 3.

Comparative Example 7

An experiment was performed as described for Comparative Example 5, except that 0.196g of an aqueous solution of hydrobromic acid (47% HBr) was further added. The results are shown in Table 3.

Comparative Example 8

An experiment was performed as described for Comparative Example 5, except that cobalt chloride hexahydrate was not used, and that 0.09g of pyridine and 0.196g of an aqueous solution of hydrobromic acid (47% HBr) were used. The results are shown in Table 3.

Table 2

| | Reaction material | Solvent | Catalyst | Reaction time (h) | Reaction temperature (℃) | Conversion (%) | m-chloroacetophenone yield(%) | Ketone selectivity (%) | 1-(3-chlorophenyl)ethanol yield (%) | m-chlorobenzoic acid yield (%) | m-chlorobenzaldehyde yield (%) |
|---|---|---|---|---|---|---|---|---|---|---|---|
| Example 1 | mCEB* 80g | Nil | $CoCl_2 6H_2O$ 0.0135g<br>Pyridine 0.0045g | 10<br>20 | 120<br>↑ | 37.7<br>59.9 | 25.0<br>44.9 | 66<br>75 | 6.2<br>4.8 | 0.9<br>1.5 | 0.2<br>0.2 |
| Example 2 | ↑ | ↑ | $Co(OAc)_2 4H_2O$ 0.0142g<br>Pyridine 0.010g | 12<br>24 | 120<br>↑ | 45.8<br>70.0 | 30.9<br>53.8 | 67<br>77 | 3.2<br>1.9 | 1.9<br>3.0 | 0.2<br>0.1 |
| Example 3 | ↑ | ↑ | $CoCl_2 6H_2O$ 0.0135g<br>Pyridine 0.09g<br>Hydrobromic acid 0.196g | 10<br>20 | 120<br>↑ | 42.1<br>69.5 | 29.0<br>52.9 | 69<br>76 | 4.9<br>3.3 | 0.9<br>2.6 | 0.1<br>0.2 |
| Example 4 | ↑ | ↑ | $CoCl_2 6H_2O$ 0.008g<br>Pyridine 0.08g<br>Hydrobromic acid 0.17g | 11<br>18 | 140<br>↑ | 26.6<br>54.8 | 20.4<br>46.8 | 77<br>85 | 5.2<br>4.6 | 0.9<br>3.0 | 0.2<br>0.4 |
| Example 5 | ↑ | ↑ | $CoCl_2 6H_2O$ 0.0135g<br>α-picoline 0.106g<br>Hydrobromic acid 0.196g | 17 | 140 | 63.8 | 50.7 | 80 | 6.0 | 4.3 | 0.4 |
| Example 6 | ↑ | ↑ | $CoCl_2 6H_2O$ 0.0135g<br>1,10-phenanthroline 0.0113g | 15<br>20 | 120<br>↑ | 42.2<br>55.4 | 29.9<br>42.7 | 71<br>77 | 5.3<br>4.3 | 1.5<br>1.8 | 0.1<br>0.1 |
| Example 7 | ↑ | ↑ | $CeCl_3 7H_2O$ 0.0212g<br>Pyridine 0.09g<br>Hydrobromic acid 0.196g | 17 | 140 | 57.4 | 44.5 | 78 | 6.1 | 3.1 | 0.4 |
| Example 8 | ↑ | ↑ | $MnCl_2 4H_2O$ 0.0113g<br>Pyridine 0.09g<br>Hydrobromic acid 0.196g | 10<br>19 | 120<br>↑ | 43.7<br>56.7 | 28.3<br>40.1 | 65<br>71 | 7.4<br>6.3 | 1.3<br>2.4 | 0.4<br>0.4 |

mCEB: m-chloroethylbenzene

Table 3

| | Reaction material | Solvent | Catalyst | | Reaction time (h) | Reaction temperature (℃) | Conversion (%) | m-chloroacetophenone yield(%) | Ketone selectivity (%) | 1-(3-chlorophenyl)ethanol yield (%) | m-chlorobenzoic acid yield (%) | m-chlorobenzaldehyde yield (%) |
|---|---|---|---|---|---|---|---|---|---|---|---|---|
| Comparative Example 1 | mCEB* 80g | Nil | $CoCl_2 6H_2O$<br>Pyridine | 0.0135g<br>0.0045g | 12<br>20 | 120<br>↑ | 19.5<br>29.3 | 10.8<br>20.5 | 55.8<br>70.0 | 7.01<br>6.43 | 4.45<br>3.01 | 0.62<br>0.42 |
| Comparative Example 2 | mCEB 40g | AcOH 120g | $CoCl_2 6H_2O$<br>Pyridine<br>Hydrobromic acid | 0.004g<br>0.04g<br>0.085g | 12<br>18 | 110<br>↑ | 8.89<br>17.6 | 6.23<br>13.7 | 70.1<br>77.8 | 1.59<br>1.64 | 0.29<br>0.61 | 0.29<br>0.40 |
| Comparative Example 3 | mCEB 40g | AcOH 120g | $CoCl_2 6H_2O$<br>Pyridine<br>Hydrobromic acid | 0.016g<br>0.16g<br>0.34g | 8<br>18 | 110<br>↑ | 10.4<br>19.9 | 6.36<br>13.8 | 61.2<br>69.3 | 2.35<br>2.78 | 0.60<br>1.92 | 0.58<br>0.95 |
| Comparative Example 4 | mCEB 14.1g | AcOH 120g | $CoBr_2 6H_2O$<br>$Co(OAc)_2 4H_2O$<br>NaOAc | 0.0872g<br>0.117g<br>0.0381g | 7 | 110 | 14.0 | 8.96 | 64.0 | 3.56 | 0.58 | 0.73 |
| Comparative Example 5 | mCEB 80g | Nil | $CoCl_2 6H_2O$ | 0.0135g | 24 | 120 | 17.8 | 13.3 | 75 | 2.9 | 0.3 | 0.4 |
| Comparative Example 6 | ↑ | ↑ | $Co(acac)H_2O$ | 0.0147g | 19 | 120 | 24.1 | 14.1 | 59 | 5.0 | 0.3 | 0.3 |
| Comparative Example 7 | ↑ | ↑ | $CoCl_2 6H_2O$<br>Hydrobromic acid | 0.0135g<br>0.196g | 24 | 120 | 21.2 | 12.9 | 61 | 4.8 | 0.6 | 0.3 |
| Comparative Example 8 | ↑ | ↑ | Pyridine<br>Hydrobromic acid | 0.09g<br>0.196g | 23 | 120 | 34.3 | 23.4 | 68 | 5.9 | 0.8 | 0.5 |

mCEB: m-chloroethylbenzene

Example 9

An experiment was performed as described for Example 4, except that 80 g of m-chloropropylbenzene was used instead of m-chloroethylbenzene (mCEB), and that the reaction temperature was changed from 120°C to 130°C. The m-chloropropylbenzene conversion, m-chloropropiophenone yield, 1-(3-chlorophenyl)-propanol yield, m-chlorobenzoic acid yield and m-chlorobenzaldehyde yield were calculated respectively from the following formulae.

m-chloropropylbenzene conversion (%) =
(Moles of reacting m-chloropropylbenzene)/(Moles of m-chloropropylbenzene in raw materials) x 100

m-chloropropiophenone yield (%) =
(Moles of produced m-chloropropiophenone)/(Moles of m-chloropropylbenzene in raw materials) x 100

1-(3-chlorophenyl)propanol yield (%) =
(Moles of produced 1-(3-chlorophenyl)propanol)/(Moles of m-chloropropylbenzene in raw materials) x 100

m-chlorobenzoic acid yield (%) =
(Moles of produced m-chlorobenzoic acid)/(Moles of m-chloropropylbenzene in raw materials) x 100

m-chlorobenzaldehyde yield (%) =
(Moles of produced m-chlorobenzaldehyde)/(Moles of m-chloropropylbenzene in raw materials) x 100

The reaction results are shown in Table 4.

Comparative Example 9

An experiment was performed as described for Comparative Example 3, except that 40 g of m-chloropropylbenzene (mCPB) was used instead of 40 g of m-chloroethylbenzene (mCEB). The results are shown in Table 4.

Comparative Example 10

An experiment was performed as described for Comparative Example 4, except that 15.6 g of m-chloropropylbenzene (mCPB) was used instead of 14.1 g of m-chloroethylbenzene (mCEB). The results are shown in Table 4.

Example 10

The reaction solution obtained in Example 1 was fractionally distilled using a packed reduced pressure fractional distiller to obtain m-chloroacetophenone. The obtained m-chloroacetophenone (purity 98%) contained, by weight of the total weight of the composition, m-chlorostyrene (0.3%), m-chlorobenzaldehyde (0.3%), 1-(3-chlorophenyl)ethanol (1.0%) and 1-(3-chlorophenyl)-1-chloroethane (0.4%).

Example 11

A glass three-neck flask with a reflux condenser and a Dean-Stark device was charged with 80 g of pDEB, and into the flask, 0.0142 g of cobalt chloride hexahydrate, 0.0189 g of pyridine and 0.041 g of 47% hydrogen bromide water were supplied. With stirring by stirring blades, air was blown in at atmospheric pressure at 150 m/min. In succession, the flask was heated by an oil bath to 120°C, to initiate reaction. The water produced in the reaction was removed from the reaction solution using the Dean-Stark device. After reaction for a predetermined period of time, the reaction solution was analyzed by gas chromatography and high speed liquid chromatography. The pDEB conversion and p-diacetylbenzene (pDAB) yield were calculated from the following formulae.

p-diethylbenzene conversion (%) =
(Moles of reacting p-diethylbenzene)/(Moles of p-diethylbenzene in raw materials) x 100

$$\text{p-diacetylbenzene yield (\%)} = \text{(Moles of produced p-diacetylbenzene)/(Moles of p-diethylbenzene in raw materials)} \times 100$$

The results are shown in Table 5.

Comparative Example 11

An experiment was performed as described for Example 11, except that pyridine and hydrogen bromide were not supplied. The results are shown in Table 5.

Example 12

An experiment was performed as described for Example 11, except that m-diethylbenzene was used instead of p-diethylbenzene.

The mDEB conversion and m-diacetylbenzene (mDAB) yield were calculated from the following formulae.

$$\text{m-diethylbenzene conversion (\%)} = \text{(Moles of reacting m-diethylbenzene)/(Moles of m-diethylbenzene in raw materials)} \times 100$$

$$\text{m-diacetylbenzene yield (\%)} = \text{(Moles of produced m-diacetylbenzene)/(Moles of m-diethylbenzene in raw materials)} \times 100$$

The results are shown in. Table 5.

Comparative Example 12

An experiment was performed as described for Comparative Example 11, except that m-diethylbenzene was used instead of p-diethylbenzene. The results are shown in Table 5.

Table 4

| | Reaction material | Solvent | Catalyst | Reaction time (h) | Reaction temperature (℃) | Conversion (%) | m-chloropropiophenone yield(%) | Ketone selectivity%) | 1-(3-chlorophenyl)propanol yield(%) | m-chlorobenzoic acid yield(%) | m-chlorobenzaldehyde yield(%) |
|---|---|---|---|---|---|---|---|---|---|---|---|
| Example 9 | mCPB* 80g | Nil | $CoCl_2 4H_2O$ 0.008g<br>Pyridine 0.08g<br>Hydrobromic acid 0.17g | 5<br>12 | 130<br>↑ | 10.5<br>19.6 | 4.41<br>7.94 | 42.0<br>40.5 | 2.23<br>3.10 | 2.08<br>6.10 | 1.74<br>2.44 |
| Comparative Example 9 | mCPB 40g | AcOH 120g | $CoCl_2 4H_2O$ 0.016g<br>Pyridine 0.16g<br>Hydrobromic acid 0.34g | 7<br>12 | 110<br>↑ | 2.97<br>10.0 | 0.93<br>3.40 | 31.2<br>33.9 | 1.01<br>1.81 | 0<br>1.87 | 1.03<br>2.94 |
| Comparative Example 10 | mCPB 15.6g | AcOH 120g | $CoBr_2 6H_2O$ 0.0872g<br>$Co(OAc)_2 4H_2O$ 0.117g<br>NaOAc 0.0381g | 7<br>12 | 110<br>↑ | 11.0<br>19.8 | 3.58<br>6.77 | 32.6<br>34.2 | 1.82<br>2.35 | 1.40<br>3.82 | 4.20<br>5.80 |

mCPB: m-chloro-n-propylbenzene

Table 5

| | Reaction material | Reaction time (h) | Reaction temperature (℃) | Diethylbenzene conversion(%) | Diacetylbenzene yield(%) | Selectivity (%) |
|---|---|---|---|---|---|---|
| Example 11 | pDEB | 40 | 120 | 87.4 | 22.0 | 25.2 |
| Comparative Example 11 | pDEB | 40 | 120 | 27.3 | 4.78 | 17.5 |
| Example 12 | mDEB | 40 | 120 | 86.4 | 21.9 | 25.4 |
| Comparative Example 12 | mDEB | 40 | 120 | 30.2 | 5.70 | 18.9 |

pDEB: p-diethylbenzene

Industrial Applicability

Since by a method of this invention an aromatic compound having an R-$CH_2$ group ( R denotes an alkyl group, aryl group, allyl group or aralkyl group) can be converted efficiently into the corresponding aromatic ketone, it is very advantageous as an industrial method of producing an aromatic ketone by air oxidation in liquid phase.

**Claims**

1. A method of producing an aromatic ketone, comprising subjecting an aromatic compound having an R-$CH_2$ group ( R denotes an alkyl group, aryl group, allyl group or aralkyl group) as a reaction raw material to a liquid phase oxidation reaction using an oxygen-containing gas in the presence of a catalyst comprising a heavy metal compound and at least one compound selected from (1) ammonia, (2) organic basic compounds and (3) onium halides while water produced in the reaction is removed from the reaction system.

2. A method according to claim 1, wherein the liquid phase oxidation reaction is effected in a rection system substantially free from any solvent or with 50 parts by weight or less of a solvent per 100 parts by weight of the reaction raw material.

3. A method according to claim 2, wherein the liquid phase oxidation reaction is effected in a solvent-less system.

4. A method according to any preceding claim, wherein the aromatic compound as the reaction raw material is represented by the following formula (I)

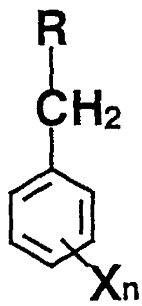

(where X is a substituent; n is zero or an integer of 1 to 5 and R is as defined in claim 1).

5. A method according to claim 4, wherein the substituent X is at least one moiety selected from alkyl, aryl, aralkyl, allyl, halogen, haloalkyl, cyano, amino, N-alkylamino, N,N-dialkylamino, alkoxyl, sulfonic acid, hydroxyl, hydroxymethyl, formyl, nitro, acyloxy, acyl, amido, $SR^1$, $SOR^1$, $SO_2R^2$, $CH_2OR^1$, $CH(OR^1)(OR^2)$, $C(OR^1)(OR^2)(OR^3)$ and $COCOR^1$ ($R^1$, $R^2$ and $R^3$ denote, respectively independently, an alkyl group, aryl group, allyl group or aralkyl group or a hydrogen atom).

6. A method according to any one of claims 1 to 3, wherein the aromatic compound as the reaction raw material is an aromatic compound having two or more R-$CH_2$ groups ( R denotes an alkyl group, aryl group, allyl group or aralkyl group).

7. A method according to claim 6, wherein the aromatic compound with two or more R-$CH_2$ groups is diethylbenzene.

8. A method according to any preceding claim, wherein the reaction is effected in the presence of (1) ammonia or (2) organic basic compound and a halogen compound is additionally present.

9. A method according to claim 8, wherein the halogen compound is a bromine compound.

10. A method according to any one of claims 1 to 9, wherein the heavy metal compound contains at least one metal selected from cobalt, manganese, cerium, vanadium, chromium, tungsten, molybdenum, ruthenium, copper and iron.

11. A method according to any one of claims 1 to 7, wherein the catalyst includes an organic basic compound which is an amine compound.

12. A method according to claim 11, wherein the amine compound is a tertiary amine.

13. A method according to any one of claims 1 to 7, wherein the catalyst includes an onium halide which is at least one onium halide component selected from ammonium halides, pyridinium halides, oxonium halides, sulfonium halides and phosphonium halides.

14. An aromatic ketone composition comprising an aromatic ketone and at least one compound selected from R-CHY group substituted benzenes represented by the following formula (II)

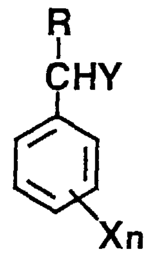

(where X is a substituent group; n is zero or an integer of 1 to 5; Y is a hydroxyl group or halogen atom and R is as defined in claim 1),
vinylbenzenes represented by the following formula (III)

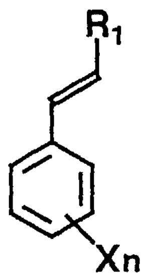

(where X is a substituent; n is zero or an integer of 1 to 5; and $R_1$ is a hydrogen atom, alkyl group, aryl group, allyl group or aralkyl group), and
benzaldehydes represented by the following formula (IV)

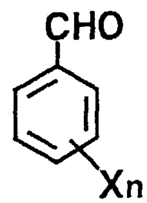

(where X is a substituent; n is zero or an integer of 1 to 5), each compound of (II), (III) and (IV) present being present individually in an amount of from 10 ppm to 3 wt% by weight of the total weight of the composition.

15. An aromatic ketone composition according to claim 14, wherein a vinylbenzene represented by the formula (III) is present in an amount of 10 ppm to 3 wt%.

16. An aromatic ketone composition according to claim 14, wherein a benzaldehyde represented by the formula (IV) is present in an amount of 10 ppm to 3 wt%.